# EUROPÄISCHE PATENTSCHRIFT

(11) **EP 1 198 555 B1**
(45) Veröffentlichungstag und Bekanntmachung des Hinweises auf die Patenterteilung: **12.04.2006**
(21) Anmeldenummer: 00952988.4
(22) Anmeldetag: 01.07.2000
(51) Int. Cl.: C12M 3/06

(54) **VORRICHTUNG ZUM ZÜCHTEN UND/ODER BEHANDELN VON ZELLEN**
METHOD FOR CULTURING AND/OR TREATING CELLS
DISPOSITIF DE CULTURE ET DE TRAITEMENT DE CELLULES

(30) Priorität: 29.07.1999 DE 19935643
(43) Veröffentlichungstag der Anmeldung: 24.04.2002
(73) Patentinhaber: Bader, Augustinus, 31275 Lehrte (DE)
(72) Erfinder: Bader, Augustinus, 31275 Lehrte (DE)
(86) Internationale Anmeldenummer: PCT/EP2000/006153
(87) Internationale Veröffentlichungsnummer: WO 2001/009282

(56) Entgegenhaltungen:
- DE-A- 19 719 751

## Beschreibung

Die Erfindung betrifft eine Vorrichtung zum Züchten und/oder Behandeln von Zellen nach der im Oberbegriff von Anspruch 1 näher definierten Art. Die Erfindung betrifft auch ein Verfahren zum Züchten und Behandeln von Zellen.

In der DE 42 06 585 C2 ist eine Vorrichtung zur Massenkultur von Zellen, insbesondere von Hepatozyten, auf plattenartigen Zellkulturträgern beschrieben, wobei wenigstens ein Teil der Oberflächen der Zellkulturträger gasdurchlässig ist. In das Innere der Zellkulturträger ist Sauerstoff einleitbar und auf dem Zellkulturträger ist eine die Zellen bedeckende Kollagenschicht aufgetragen. Direkt oder mit geringem Abstand über der Kollagenschicht ist der nächste Zellkulturträger angeordnet. In die Kollagenschicht oder in den Zwischenraum zwischen der Kollagenschicht und dem nächsten Zellkulturträger ist Kulturmedium einleitbar. Die Vorrichtung ist sandwichartig aufgebaut. Nachteilig bei dieser Vorrichtung ist, daß stets genau definierte Zellenkammern mit bestimmten Volumina vorhanden sind. Darüber hinaus ist es schwierig die Zellen in den Zellenkammern zu beobachten, um eventuelle Veränderungen bzw. Entwicklungszustände festzustellen.

In der DE 42 22 345 A1 ist ein Verfahren zum Züchten einer Zellart im Cokulturverfahren mit Leberzellen beschrieben, wobei eine Kultivierung von Leberzellen auf einem Träger im Sandwichverfahren erfolgt. Zwischen den Leberzellen und dem Träger ist eine erste Matrixschicht zur Verankerung der Leberzellen angeordnet und über den Leberzellen befindet sich eine zweite Matrixschicht.

Die WO 95/17526 beschreibt ein Verfahren zum Züchten von Haut nach einem sogenannten "air Lift"-Verfahren. Dabei wird Gas oder sterile Luft in eine Zellwachstumskammer gebracht. Die Zellen sitzen dabei auf einer semipermeablen Membran. Basalzellen, die auf der semipermeablen Membran liegen, werden über die Mediumkammer mit Nährstoffen aufgrund Diffusion versorgt. Die Basalzellen wachsen zur Luftphase hin mehrschichtig. Nachteilig ist dabei jedoch, daß bei dem vorbekannten Verfahren die Hautstruktur "auf dem Kopf" liegt. Dies bedeutet, daß andere Zelltypen, wie z.B. Bindegewebszellen (Fibroblasten) entweder zuerst ausgesät und abgewartet werden muß oder die Haut muß mechanisch angehoben werden. Auf diese Weise kann es jedoch zu einer Zerstörung der empfindlichen Strukturen kommen.

Die DE 197 19 751 A1 betrifft eine Vorrichtung zum Züchten und/oder Behandeln von Zellen, wobei auf einem gasdurchlässigen Träger eine Zellenkammer angeordnet ist. Die Zellenkammer ist durch eine gaspermeable, nicht flüssigkeitsdurchlässige Folie gebildet, die auf den Träger aufgelegt ist, und durch eine zweite mikroporöse Folie, die über der ersten Folie angeordnet oder mit dieser einstückig ist. Wenigstens eine der beiden Folien ist derart elastisch, daß das Endvolumen in der Zellenkammer ein Vielfaches seines Ausgangsvolumens einnehmen kann. Die Zellenkammer ist mit wenigstens einer Zu- und/oder Ablaufleitung versehen. Diese vorbekannte Vorrichtung ist insbesondere für ein Massenkultursystem geeignet. Darüber hinaus läßt sie sich den jeweiligen Anforderungen anpassen, denn man kann mit einem sehr kleinen Volumen und einer geringen Zellenzahl beginnen, die in der Zellenkammer behandelt oder kultiviert werden, und dann im Laufe der Behandlung das Anfangsvolumen auf ein Vielfaches vergrößern. Dabei können auch ein oder mehrere Einheiten sandwichartig übereinander in einem konventionellen Wärmeschrank gestellt werden.

Der vorliegenden Erfindung liegt die Aufgabe zugrunde eine Vorrichtung nach der DE 197 19 751 A1 weiter zu verbessern, insbesondere eine weitere Vereinfachung und eine noch größere Variabilität der Vorrichtung.

Erfindungsgemäß wird diese Aufgabe durch die in Anspruch 1 genannten Merkmale gelöst.

Erfindungsgemäß sind nunmehr der Träger, der auch als Rahmen bzw. rahmenartig ausgebildet sein kann oder die Stabilitätsstrukturen selbst formbar, und zwar sowohl vor als auch während des Kultivierverfahrens.

Ein erfindungsgemäßes Verfahren ist in Anspruch 37 angegeben.

Eines der wesentlichen Vorteile der erfindungsgemäßen Vorrichtung und des erfindungsgemäßen Verfahrens besteht darin, dass man auf diese Weise einen sehr flachen Zellkulturraum mit minimaler Schichtdicke erhält, wobei die Außenwände des Zellkulturraumes wenigstens teilweise gaspermeabel sind. Durch die Formbarkeit des Trägers oder des Zellkulturraumes wird eine hohe Variabilität der Vorrichtung bei einer einfachen Ausgestaltung erreicht. Ein weiterer Vorteil besteht darin, dass durch die Geometrie der Vorrichtung eine Formbarkeit des Zellkulturraumes in nahezu beliebiger Weise möglich wird.

Die in den Unteransprüchen aufgezeigten Rahmen und Stabilitätsstrukturen für die Bildung des Zellkulturraumes und der Zellkulturen ermöglichen eine noch weitergehende Formbarkeit, und zwar sowohl vor als auch während der Behandlung der Zellen.

Ein weiterer wesentlicher Vorteil der erfindungsgemäßen Lösung besteht darin, dass man die gesamte Einheit mit Zellen in einer definierten Form kontrolliert einfrieren und lagern kann. Auf diese Weise kann die erfindungsgemäße Vorrichtung auch als Transportsystem verwendet werden.

Wie ersichtlich, ist eines der wesentlichen Unterschiede im Vergleich zum Stand der Technik, wobei nur flexible Zellkulturräume bzw. Folien vorhanden sind, nunmehr neben der Möglichkeit einer Flexibilität auch eine Formbarkeit bzw. Modellierbarkeit des Zellkulturraumes bzw. dessen Geometrie zu erreichen. Auf diese Weise lassen sich praktisch beliebige Formen und Gestaltungen für den Zellkulturraum und damit praxisnahe Ergebnisse schaffen.

Wenn in einer erfindungsgemäßen Ausgestaltung vorgesehen ist, daß der Zellkulturraum in Dünnschichtstruktur ausgebildet ist, dann sind, insbesondere zu Beginn der Kultivierung, sehr geringe Sauerstoffdiffusionswege vorhanden.

Vorteilhafte Ausgestaltungen und Weiterbildungen ergeben sich aus den Unteransprüchen und aus den nachfolgend anhand der Zeichnung prinzipmäßig beschriebenen Ausführungsbeispielen.

Es zeigt:
- Fig. 1: in schematischer Darstellung eine Draufsicht auf eine Trägerplatte der erfindungsgemäßen Vorrichtung;
- Fig. 2: einen Schnitt nach der Linie II-II der Fig. 1 mit einer flexiblen Folie und einer Abdeckplatte;
- Fig. 3: eine um 180° gedrehte Anordnung der Vorrichtung nach der Fig. 2 (ohne Abdeckplatte);
- Fig. 4: in perspektivischer Ansicht eine rahmenartige Trägerplatte;
- Fig. 5: einen Querschnitt nach der Linie V-V der Fig. 4 in vergrößerter Darstellung;
- Fig. 6: Prinzipdarstellung eines Bioreaktors;
- Fig. 7: eine in Kreisform gebogene Trägerplatte in perspektivischer Darstellung;
- Fig. 8: eine in Spiralform gebogene Trägerplatte in perspektivischer Darstellung;
- Fig. 9: eine Draufsicht auf eine Struktur als Herzklappe;
- Fig. 10: eine Struktur als Herzklappe im Längsschnitt; und
- Fig. 11: eine Struktur zur Bildung einer Herzklappe in einem Behälter.

In einer grundsätzlichen Ausgestaltung weist die Vorrichtung eine formbare Trägerplatte als Träger 1 auf, die vorzugsweise aus Kunststoff besteht. Auf die Trägerplatte 1 wird eine gaspermeable und flexible Kunststoffolie 2 aufgebracht, welche an den Rändern dicht mit der Trägerplatte 1 verbunden wird.

Die Trägerplatte 1 ist an einer Stirnseite im Randbereich mit einem Zulaufanschluß 4 versehen, durch den z.B. Nährmedium zugeführt wird, welches aus einer Innenbohrung 9, die mit dem Zulaufanschluß 4 verbunden ist, in eine entlang einer Seitenwand der Trägerplatte 1 verlaufende Zulaufrinne 5 einmündet. In der in die Oberfläche der Trägerplatte 1 eingebrachte Zulaufrinne 5 fließt das Nährmedium seitlich in Richtung der Stirnseite, die der Zulaufseite gegenüberliegt. Auf dieser Stirnseite mündet die Zulaufrinne 5 in eine Querrinne 6 ein, die sich wenigstens annähernd über die gesamte Breite der Trägerplatte 1 erstreckt. Von der Querrinne 6 aus führen schräg nach oben verlaufende Zungen 7 zur Oberseite der Trägerplatte 1.

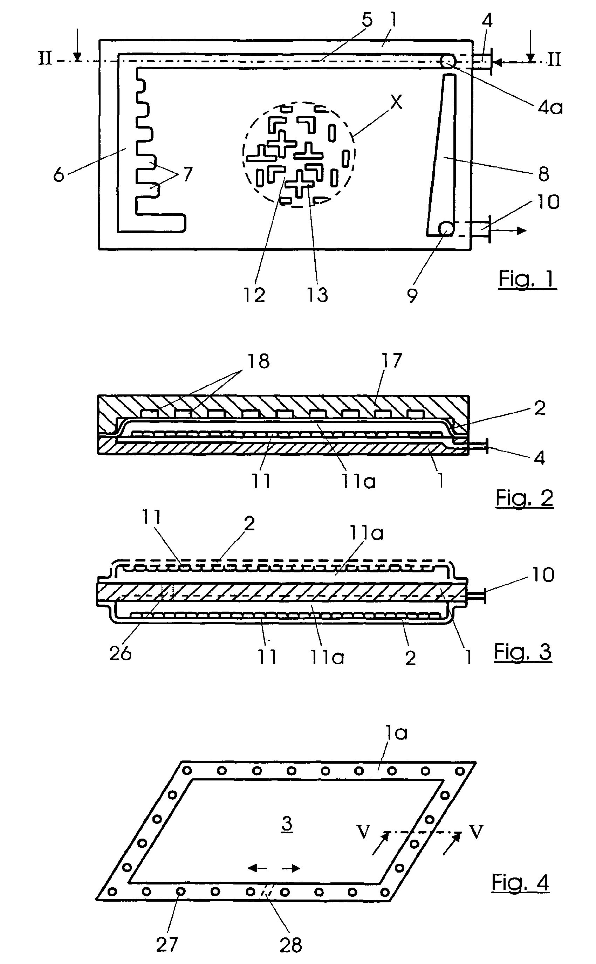

Wie aus der Fig. 1 ersichtlich ist, nimmt die Länge und gegebenenfalls auch die Breite der Zungen 7 mit zunehmendem Abstand von der Zulaufrinne 5 zu, damit das Nährmedium gleichmäßig auf der Oberfläche der Trägerplatte 1 verteilt wird und sich auf dieser Weise ein gleichmäßiger Strom in Richtung zu einer ebenfalls quer verlaufenden Auslaufrinne 8 bewegt. Um auch hier einen gleichmäßigen Fluß zu erzeugen und einen entsprechend gleichmäßigen Abfluß kann die Auslaufrinne 8 ebenfalls mit spiegelbildlich dazu liegenden Zungen 7 versehen sein. Alternativ ist es auch möglich - wie in der Fig. 1 dargestellt - eine kontinuierlich sich in Richtung zu einer Auslaufbohrung 9 sich erweiternde Querrinne 8 vorzusehen. Die Auslaufbohrung 9 ist über eine Bohrung im Inneren der Trägerplatte 1 mit einem Ablaufstutzen 10 verbunden, der an die Trägerplatte 1 angesetzt ist. Die Form der beiden Querrinnen 6 und 8 und der Verlauf der Zungen 7 ist nur beispielsweise angegeben. Wesentlich ist lediglich, daß sich über die gesamte Breite der Trägerplatte 1 ein gleichmäßiger und kontinuierlicher Fluß bzw. Strom des Nährmediums ergibt.

Die Verbindung der flexiblen Folie 2 mit der Trägerplatte 1 an den Rändern kann auf beliebige Weise, z.B. durch Klemmen, Kleben, Verschweißen oder Verschrauben erfolgen. Zusammen mit dem Nährmedium oder auch einer anderen Trägerflüssigkeit kann eine Zellkultur 11 in einem Zellkulturraum 11a, der durch die Trägerfolie 3 und die Zellkulturfolie 2 als Dünnschichtstruktur ausgebildet ist, auf die Trägerplatte 1 gebracht werden.

Selbstverständlich ist es auch möglich die Zellkultur 11 vorher auf der Trägerplatte 1 zu verteilen, wobei diese ebenfalls über den Zulaufanschluß 4 eingebracht werden kann. Die Zellkultur 11 setzt sich entsprechend in der Fig. 2 dargestellten Ausführungsform direkt auf der Oberfläche der Trägerplatte 1 ab. Zur Vermeidung von Shearstreß bzw. Überströmungsstreß kann die Oberfläche der Trägerplatte 1 strukturiert sein und zwar derart, daß die Zellen sich in kleinen Vertiefungen 12 der Oberfläche festsetzen. Hierzu kann die Oberfläche der Trägerplatte 1 z.B. schachbrettartig strukturiert sein, mit entsprechenden Vertiefungen 12 und bis zur Oberfläche der Trägerplatte 1 hochgezogenen Seitenwänden 13. In der in dem gestrichelten Kreis X dargestellten Vergrößerung in der Fig. 1 ist diese Struktur ersichtlich. Um eine entsprechende Verbindung zwischen den einzelnen Vertiefungen 12 und damit der Zellen untereinander zu schaffen, können die Wände 13 auch mit Durchbrechungen versehen werden, wobei sich eine Art Labyrinth ergibt. Auch die Folien an sich können profiliert sein, z.B. schachbrettartig, oder Glasfasern oder ein anderes Material enthalten, die eine bestimmte Struktur definieren, die eventuell durch Schmelzprozesse entsprechend verändert werden.

Aus der Fig. 4 ist ersichtlich, daß die Trägerplatte 1 auch nur aus einem Rahmen 1a bestehen kann, mit welchem die flexible Folie 2 verbunden wird. Unter der flexiblen Folie 2 und ebenfalls mit der rahmenartigen Trägerplatte 1a verbunden kann der Raum zwischen dem Rahmen durch eine Trägerfolie 3, die die Außenfolie bildet, ausgefüllt sein. Die Trägerfolie 3 kann, muß jedoch nicht unbedingt, flexibel und/oder gaspermeabel sein. Die Sauerstoffzufuhr zu der Zellkultur 11 erfolgt sowohl bei dem Ausführungsbeispiel nach der Fig. 2 als auch bei dem nach der Fig. 4 über die elastische Folie 2.

Der Rahmen 1a kann mit einer Vielzahl von Noppen 27 versehen sein, die zum Einspannen einer aufgebrachten flexiblen Folie dienen bzw. zu deren Befestigung. Hierzu kann der Rahmen 1a auch - ähnlich wie zwei Dia-Rahmenhälften - konzipiert sein, wobei in diesem Fall flexible Folien 2 zwischen zwei Rahmen 1a eingelegt und gespannt werden.

Der Rahmen 1a kann auch als eine Art Spannrahmen oder als Spannfeder ausgebildet sein. Durch einen Spannrahmen wird erreicht, daß die mit den Rahmen verbundenen Folien 2 durch diese gespannt werden, womit ein Durchhängen bzw. eine sackartige Ausbildung vermieden wird. Aufgrund der Elastizität der Folien 2 wächst dann während der Kultur der Zellkulturraum 11a entsprechend. In der Fig. 4 ist die Ausgestaltung eines entsprechend offenen Spannrahmens durch einen Schlitz 28 auf einer Seite angedeutet. Der Rahmen 1a ist dabei so ausgebildet, daß in Pfeilrichtung verlaufende Kräfte zum Spannen der dazwischenliegenden Folien 2 entstehen. Anstelle der rechteckigen Ausgestaltung wird man in diesem Falle gegebenenfalls auch einen offenen runden Spannrahmen verwenden, durch den eine allseitige Spannung der Folien 2 leichter erreicht wird.

Die Noppen 27 können auch als Löcher ausgebildet sein, in denen dann die Folien 2 zum Spannen versenkt und entsprechend geklemmt werden.

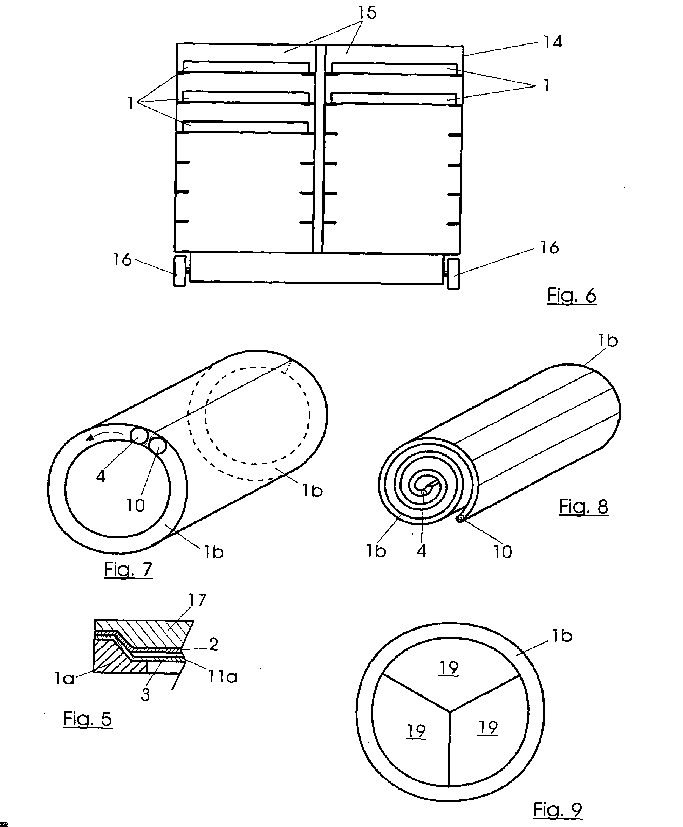

Die Fig. 5 zeigt einen Querschnitt nach der Linie V-V der Fig. 4 in vergrößerter Darstellung (insbesondere der Folien). Wie ersichtlich ist dabei der Rahmen 1a bzw. eine Leiste derart angeschrägt, daß diese nach innen abfällt. Durch diese Abschrägung werden Toträume im Zellkulturraum 11a vermieden, wenn die Folien 2 und gegebenenfalls die Folie 3 entsprechend aufgelegt werden. In diesem Falle kann man für eine exakte Führung bzw. Anlage eine in der Fig. 2 dargestellte Abdeckplatte 17 über die Folie 2 aufbringen, wobei die Abdeckplatte 17 eine entsprechend zu der Abschrägung des Rahmens 1a komplementäre Form aufweist. Die Aufgabe der Abdeckplatte 17 und deren Form wird nachfolgend noch näher beschrieben.

Die Fig. 3 zeigt eine umgekehrte Anordnung zur Zucht bzw. Behandlung einer Zellkultur 11 derart, daß sich in diesem Falle die Trägerplatte 1 auf der Oberseite befindet und die elastische Folie 2 darunter hängt. Da die Zellen der Zellkultur 11 in Richtung der Schwerkraft sedimentieren, liegen sie in diesem Falle auf der Innenseite der elastischen Folie 2 an. Im Bedarfsfalle kann dabei die elastische Folie 2 ebenfalls im Sinne der Oberflächengestaltung der Trägerplatte 1 nach der Fig. 1 strukturiert sein. Durch die Oberflächenstrukturierung erfolgt der Fluß des Nährmediums über die sich in den Vertiefungen 12 befindenden Zellen in Form eines feinen Filmes. Die elastische Folie in der Anordnung nach der Fig. 3 kann z.B. zur Ausbildung einer wabenartigen Struktur entsprechend geätzt sein. Die Verbindung der beiden Folien 2 und 3 mit der rahmenartigen Trägerplatte 1a kann z.B. durch Verschrauben oder Klammern, vorzugsweise mit einer entsprechenden Dichtung als Zwischenlage, erfolgen.

Aus der Fig. 3 ist auch in gestrichelter Darstellung ersichtlich, daß man eine Einheit an einer Trägerplatte 1 auch spiegeln kann, womit eine einzige Trägerplatte 1 für die Bildung von zwei Zellkulturräumen 11a verwendet werden kann. Dabei können die beiden Einheiten separat voneinander betrieben werden oder sie können durch ein oder mehrere Durchbrüche 26 miteinander kommunizieren. In diesem Falle kann gegebenenfalls ein gemeinsamer Zulauf und ein gemeinsamer Ablauf vorgesehen sein. Die Zellen können dabei sowohl auf der Innenseite der Zellkulturfolie 2 als auch auf der Trägerplatte 1 angebracht werden. Auf diese Weise wird eine deutliche Material- und Kosteneinsparung erreicht.

Selbstverständlich kann die Überströmung der Trägerplatte 1 mit Nährmedium auch in einfacher Weise von der Zuströmseite mit dem Zulaufanschluß 4 zu der gegenüberliegenden Seite direkt erfolgen und dann dort abgeführt werden. In diesem Falle würde die Zulaufrinne 5 entfallen und lediglich anstelle der den Ablauf bildenden Querrinne 8 diese dann als Zulaufrinne ausgebildet sein und auf der anderen Seite eine entsprechende Ablaufrinne in der Trägerplatte 1 eingebracht sein. Aus praktischen Gründen wird man jedoch die in der Fig. 1 gewählte Strömungsrichtung vorsehen, da in diesem Falle sämtliche Zu- und Ableitungen, welche im allgemeinen in Schlauchform vorgesehen sind, von einer Seite aus erfolgen und damit komplett von einer Seite aus zugänglich sind. Diese Ausgestaltung wird man insbesondere dann vorsehen, wenn - wie in der Fig. 6 dargestellt - mehrere Vorrichtungen in einer Art Bioreaktor 14, z.B. in Form eines Hochregales, behandelt werden. Der Bioreaktor 14 besitzt hierzu eine Vielzahl von entsprechend übereinander angeordneten Einschubfächern 15, in die die Vorrichtungen nach den Figuren 1 bis 4 eingeschoben werden können. Alle Zu- und Abläufe befinden sich dann dabei auf einer Seite. Die einzelnen Module können auch direkt miteinander verbunden werden.

In dem Bioreaktor 14 können - wie ersichtlich - auch mehrere Reihen von Einschubfächern 15 nebeneinander angeordnet sein. Im allgemeinen wird man den Bioreaktor 14 auch mit Rädern 16 für eine leichte Transportierbarkeit versehen.

Anhand der Fig. 2 ist als weitere Möglichkeit zur Behandlung einer Zellkultur 11 die Abdeckplatte 17 erläutert. Die Abdeckplatte 17 wird auf die Folie 2 aufgesetzt. Durch die Abdeckplatte 17 kann das Volumen minimiert werden. Die Abstände können auch flexibel eingestellt werden. Dies ist insbesondere in der Vorbereitungsphase, z.B. in den ersten 48 Stunden, von Vorteil, wenn man die Zellkultur 11 in Ruhe lassen möchte. Auf diese Weise entsteht kein zu großer Totraum über dem System und es erhöht sich gleichzeitig auch der Diffusionsaustausch. Wie ersichtlich ist die Abdeckplatte 17 auch mit Kanälen 18 oder einen Wabenstruktur versehen, welche an den seitlichen Rändern offen ist, damit eine Luft- bzw. Sauerstoffzuführung über die Kanäle 18 und von da aus durch die luftdurchlässige Folie 2 zu der Zellkultur 11 gegeben ist. Eine Verbindung der Abdeckplatte 17 mit der Trägerplatte 1 und auch eine dichte Verbindung der Folie 2 und - gegebenenfalls der Trägerfolie 3 - an den Rändern kann durch nicht dargestellte Clipse oder eine entsprechende Schraubverbindung erfolgen.

Die Abdeckplatte 17 nach der Fig. 2 kann auch derart ausgebildet werden, daß die Kanäle 18 auf einer Zufuhrseite mit einem Sauerstoffträger (nicht dargestellt) verbunden und an den übrigen Rändern geschlossen sind. Auf diese Weise läßt sich eine eigene Sauerstoffversorgung für die Zellen schaffen. Der Vorteil einer derartigen autarken Vorrichtung ist, daß man dann Luft mit einer bestimmten Temperatur und/oder Feuchtigkeit zuführen kann und auf diese Weise eine eigene "Biosphäre" schaffen kann. Ein weiterer Vorteil dieser Ausgestaltung besteht darin, daß man im Bedarfsfalle auch Luft mit Überdruck eingeben kann, womit man den Massentransfer und den Partialdruck erhöhen kann. Grundsätzlich wäre es selbstverständlich auch möglich die Sauerstoffkonzentration entsprechend zu erhöhen, aber bestimmte Zellkulturen reagieren negativ auf einen zu hohen Sauerstoffgehalt. Eine Behandlung unter einem erhöhten Druck kann sich jedoch - in Abhängigkeit von den zu behandelnden Zellkulturen - positiv auswirken.

Eine weitere Ausgestaltung kann darin bestehen, daß man in die Zellkammer 11a eine Netzstruktur gibt. Knochenmark wächst bekanntlich im Knochen und um das Ergebnis zu verbessern kann man in den Innenraum knochenähnliche Strukturen oder entsprechend eine Art Netzstruktur oder eine extrazelluläre Matrix geben, wodurch die Zellen eine Art Mikroumgebung erhalten, die der normalen Umgebung nahekommt. Eine derartige Netzstruktur kann man frei in den Zellkammerraum 11a eingeben oder diese bereits in die Folie 2 oder die Trägerfolie 3 einarbeiten. So könnte man z.B. Kalkstrukturen (Tricalziumphosphat) in die Zellkammer 11a eingeben.

Damit die elastische Folie 2 und gegebenenfalls bei Verwendung der Trägerfolie 3 die Folien nicht reißen, können sie im Randbereich entsprechend verstärkt sein. In diese Verstärkung können gegebenenfalls auch Zulauf- und/oder Ablaufanschlüsse eingeschweißt werden, welche dann den Zulaufanschluß 4 und den Rücklaufanschluß 10 der Trägerplatte 1 ersetzen. Bei Verwendung einer rahmenartigen Trägerplatte 1a, wie in der Fig. 4 dargestellt, ergeben sich mit der Trägerfolie 3 und der elastischen Folie 2 Zellkulturräume 11a. Ebenso wie die Ausgestaltung nach den Figuren 1 bis 3 lassen sich rahmenartige Trägerplatten 1a mit den daran befestigten Folien 2 und 3 beliebig übereinander stapeln, wobei man in diesem Falle in die Folien 2 und 3 - wie vorstehend erwähnt - die Zulauf- und Ablaufanschlüsse einschweißen wird.

Die einzelnen Vorrichtungen mit ihren Trägerplatten 1 können auch in Form eines Stecksystemes zu beliebig langen Einheiten miteinander verbunden werden. In diesem Falle dienen entsprechende Bohrungen und Stifte zur Verbindung. Hierzu ist es dann lediglich erforderlich, daß man die einzelnen Zulaufanschlüsse 3 und die Ablaufanschlüsse 10 entsprechend miteinander verbindet.

Statt einer Trägerplatte 1a in Rahmenform, wie in der Fig. 4 dargestellt, kann eine rahmenartige Trägerplatte 1a auch durch einen Spannrahmen oder einen Spanndraht in beliebiger Form, z.B. in rechteckiger, quadratischer oder Kreisform ersetzt werden, mit welchem die beiden Folien 2 und 3 verbunden sind. Mit anderen Worten: Die Rahmenkonstruktion nach der Fig. 4 kann zu einem Kreis gebogen werden, wie es in der Fig. 7 prinzipmäßig dargestellt ist oder auch in eine Spiralform, wie es aus der Fig. 8 ersichtlich ist, wobei das Material hierzu formbar bzw. elastisch sein kann. Durch die Ausgestaltungen nach den Figuren 7 und 8, insbesondere der Fig. 7, ergibt sich ein flexibles System in Form einer Röhre mit einer jeweils einer Trägerplatte bzw. einem Trägerring 1b und dazwischenliegenden Folien 2 und 3 in deren Innerem sich die Zellkultur 11a befindet. Wenn dabei als Ausgangsmaterial eine rahmenartige Trägerplatte 1a gemäß Fig. 4 gewählt wird, auf der die beiden Folien 2 und 3 befestigt sind, liegen nur an beiden Stirnseiten der Röhre Versteifungsringe vor, die durch zwei nebeneinander liegende Verbindungsleisten entlang der Mantellinie miteinander verbunden sind. Ansonsten ist die Umfangswand der Röhre flexibel. Gegebenenfalls können die beiden Verbindungsleisten auch entfallen. In diesem Falle hat man nur zwei Versteifungsringe an beiden Stirnseiten.

Die in der Fig. 7 dargestellte Struktur in Ringform stellt nur eine grundsätzliche Ausgestaltung dar und läßt sich in vorteilhafter Weise aus dieser Grundform abwandeln. Ein mögliches Einsatzgebiet ist z.B. ein Herzkranzgefäß-Bypass, wobei bisher dieser Bypass von einer Beinvene des Patienten entnommen werden mußte. Nachteilig dabei ist jedoch, daß sich Venen häufig nach mehreren Jahren wieder verschließen, da diese im Bereich der arteriellen Strömungen am Herz den höheren Druck nicht aushalten.

Mit einer erfindungsgemäßen Ausführungsform nach der Fig. 7 läßt sich auf diese Weise mit den eigenen Zellen des Patienten ein Gefäß züchten, wobei zwischen die Folien 2 und 3 in den Zellkulturraum 11a Bindegewebszellen zusammen mit einem Kollagengemisch injiziert werden. Anschließend läßt man die Zellen für eine bestimmte Zeit bis zum Gebrauch wachsen.

Eine andere Einsatzmöglichkeit besteht für neuronale Zellen. Auch diese Zellen kann man in die auf diese Weise geschaffene Röhre geben, wobei jedoch in bestimmten Kulturphasen vermieden werden soll, daß sie anwachsen. Die Röhre dient dabei lediglich dazu, daß sich die neuronalen Zellen vermehren, so daß man sie anschließend wieder entnehmen und für den Nervenkranken verwenden kann.

Fig. 9 zeigt in Prinzipdarstellung in der Draufsicht eine Röhre mit drei nach innen gezogenen "Segeln" 19. Durch diese Ausgestaltung wird eine Stabilitätsstruktur geschaffen, die als Herzklappe dienen kann.

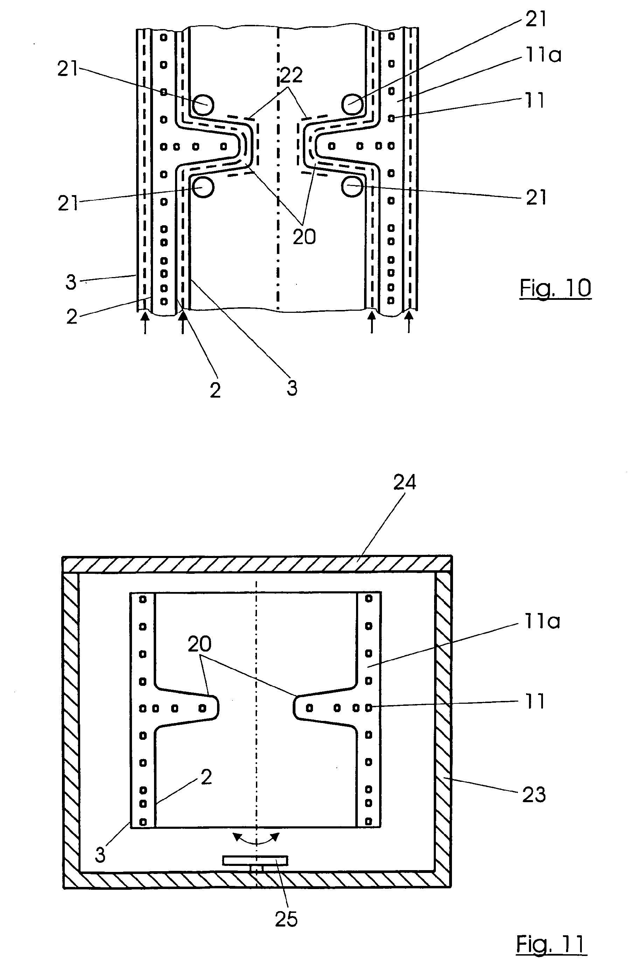

In der Fig. 10 ist eine Stabilitätsstruktur im Längsschnitt dargestellt, die eine Herzklappe bilden kann. Wie ersichtlich weist die Röhre nach innen gezogene Einstülpungen 20 auf. Je nach Verwendungszweck einer derartigen Röhre können die Einstülpungen auch so weit nach innen gehen, daß sie sich berühren bzw. daß sie sogar eine Querverbindung in dem ringförmigen Zellkulturraum 11a bilden.

Bei der Herstellung dieser Form können z.B. Stabilisierungsringe 21 über und unter den Einstülpungen 20 angeordnet werden. Zur genauen Formbestimmung können auch über die Einstülpungen 20 innenseitig Klammern 22 gesetzt werden (gestrichelt dargestellt). Die Trägerfolie 3 als Träger auf der Außenseite und auch die Trägerfolie 3 auf der Innenseite sind luftdurchlässig und formbar. Jeweils zwischen den äußeren Folien 3 und den auf geringen Abstand dazu angeordneten elastischen Folien 2 fließt Nährmedium. Die elastischen Folien 2 sind in diesem Falle zusätzlich mikroporös, so daß die Nährstoffe bzw. das Nährmedium in den innen liegenden ringarten Zellkulturraum 11 mit den darin angeordneten Zellen 11 diffundieren kann.

Der Zellkulturraum 11a, in welchem die den jeweiligen Anwendungsfall entsprechenden Zellen 11 eingebracht sind, kann auch durch eine Struktur mit mikroporösen Zellen nach Art eines "Spritzgußverfahrens" gebildet werden. In diesem Falle stellt die Struktur praktisch das Trägerteil für die Zellen 11 dar und entspricht in seiner Form der späteren Anwendungsform, z.B. einer Herzklappe oder auch einer Blase als Ersatz der eigenen Blase bei Tumorpatienten.

Im letzteren Falle hat man eine Art Doppelluftballon mit einem Ein- und Ausgang, welcher elastisch ist und in dem man in einer bestimmten Form die Zellen züchten kann. Dabei ist die jeweils äußere Folie 3 gasdurchlässig, wobei sie gegebenenfalls elastisch ist und innen befindet sich die mikroporöse Folie 2.

Die Struktur kann z.B. aus einen Gehäuse bestehen, das entweder biodegradabel ist und damit zerfällt, oder es besteht aus einem Kunststoff, der später wieder entfernt wird. Das Gehäuse sollte flexibel sein und in seiner Form der Form des zu ersetzenden oder zu bildenden Organes entsprechen. Man gibt für die Zellen auf diese Weise praktisch eine Form vor, in die man dann eine Bindegewebsmatrix bzw. Kollagenmatrix mit Zellen injiziert. Das Ganze verfestigt sich dabei und kann Netzwerke bilden, auch in dreidimensionalem Wachstum, und dabei die Matrix resorbieren.

Als weitere vorteilhafte Anwendung kann man die mikroporöse Folie 2 auch aus einem Material herstellen, das sich selbst nach einer bestimmten Zeit auflöst. Dies gilt z.B. für bestimmte Polymere, wie z.B. Polylaktid, Alkanoate oder Butyrate, welche sich z.B. nach mehreren Wochen auflösen. Wenn z.B. Bindegewebszellen in einer Matrixstruktur das Gerüst gebildet haben, dann muß die "Innenauskleidung" vieler Organe angebracht werden, was mit Endothel-Zellen gemacht wird. Diese Endothel-Zellen können z.B. aufgebracht werden, wenn sich die mikroporöse Folie 2 aufgelöst hat. In diesem Falle ist der Zellkulturraum 11a ja immer noch separat zuströmbar. Im allgemeinen wird man die Zellen 11 zusammen mit einer Matrix, z.B. Kollagen, in den Zellkulturraum 11a eingebracht, wo sie sich entsprechend vermehren können. Bei einem Ersatz für eine Herzklappe sind in diesem Falle die Endothel-Zellen innen und die Bindegewebszellen mit Muskulatur außen aufgebracht. Dies bedeutet, auf die Folie 2 sind auf der zum Röhreninneren gerichteten Seite die Endothel-Zellen aufgebracht. Ebenso ist es möglich die Folie 2 auflösbar auszugestalten oder herausnehmbar.

Dadurch, daß die Folien elastisch sind, wird für die zu kultivierenden Zellen 11 eine weitgehend natürliche Umgebung geschaffen, denn ein Herz pumpt, womit es entsprechend zu Druckwellen kommt, welche sich auf die Zellen 11 entsprechend auswirken.

Wenn fertig kultiviert ist, können die Ringe 21 und gegebenenfalls die Klammern 22 abgenommen werden, denn dann ist die Einheit selbst stabil und man kann sie an der gewünschten Stelle einsetzen.

Fig. 11 zeigt eine andere Möglichkeit eine Herzklappe oder auch eine andere Struktur herzustellen. In diesem Falle befindet sich die Röhre mit den Einstülpungen 20 in einem Behälter 23, in welchem sich Nährmedium befindet. Der Behälter, der mit nicht dargestellten Zu- und Ablauföffnung versehen ist, kann mit einem Deckel 24 versehen sein, wobei die Struktur in dem Nährmedium "schwimmt" oder darin aufgehängt ist. In diesem Falle können die beiden äußeren Folien 3 entfallen und es sind nur die mikroporösen elastischen Folien 2 vorhanden, zwischen denen sich der ringförmige Zellkulturraum 11a mit den Einstülpungen 20 befindet. In den Behälter 23 wird auch Sauerstoff bzw. Luft eingebracht, wobei für eine gleichmäßige Bewegung bzw. Verteilung ein nicht näher dargestelltes Rührorgan 25 im Behälter 23 angeordnet sein kann.

In Vereinfachung der in der Fig. 11 dargestellten Struktur bzw. des daraus resultierenden Verfahrens kann die äußere Folie 3 auch nur luftdurchlässig ausgebildet sein und wirkt als Trägerfolie. Die Zufuhr von Nährmedium aus dem Behälter erfolgt in diesem Falle dann nur von innen her über die mikroporöse innere Folie 2.

Grundsätzlich kann das Zellkultur- und Zellbehandlungsverfahren bei allen vorstehend genannten Ausführungsbeispielen in dem Zellkulturraum 11a auf folgende Weise ablaufen:

In einem ersten Schritt wird extern die Matrix bzw. das Kollagen vorbereitet. Anschließend wird die Matrix bzw. das Kollagen in den Zellkulturraum 11a injiziert. Die Strukturen bzw. der Zellkulturraum 11a kann entweder mit Zellen bereits vorbeschichtet sein oder die Zellen 11 werden zusammen mit dem Kollagen bzw. mit der Matrix direkt injiziert.

In einem nächsten Schritt erfolgt die Modellierungsphase bzw. die Zellvermehrung, wobei die Zellen 11 die Matrix entsprechend umbauen. Anschließend kann man - falls erforderlich - weitere Zelltypen zugeben, z.B. wenn sich entsprechend eine Folie aufgelöst oder man diese entfernt hat.

Ein weiterer sehr großer Vorteil der Erfindung besteht darin, daß man anschließend die fertige Struktur einfrieren kann. Dies ist bei der erfindungsgemäßen Vorrichtung und dem erfindungsgemäßen Verfahren im Unterschied zu anderen Reaktorsystemen möglich, denn es herrscht praktisch ein Dünnschichtsystem vor. Bei dem Einfrieren der Zellen und der dabei erfolgenden Kristallbildung wird Wärme in den Zellen freigesetzt. Dieser Wärmefreisetzung muß gegengekühlt werden. Bei den bekannten Reaktorsystemen ist es deshalb nicht möglich, die Zellen 11 koordiniert einzufrieren. Im vorliegenden Falle ist es jedoch aufgrund des Dünnschichtsystemes möglich, alle Zellen mehr oder weniger gleichmäßig einzufrieren, wobei der Entstehung der Wärme bei der Kristallbildung gezielt und gleichmäßig entgegengewirkt werden kann. Auf diese Weise lassen sich die Zellen 11 koordiniert einfrieren, wobei man praktisch der Wärmebildung durch eine einfache Zugasung, z.B. von Stickstoff, entgegenwirken kann.

Derart eingefrorene Strukturen können somit beliebig lange für eine spätere Verwendung vorgehalten werden und/oder auch entsprechend gekühlt in eine Klinik transportiert und dort kurzfristig nach Auftauen und gegebenenfalls nach einem nochmaligen Nachspülen, implantiert werden. Diese bedeutet, die erfindungsgemäße Vorrichtung und das Verfahren ist nicht nur ein Kultivierungssystem, sondern auch ein Transportsystem, wobei die Sterilkette nicht unterbrochen wird.

Bei einem Einsatz für Leberzellen ist auch ein extra koporaler Anschluß bzw. Verwendung möglich.

In einer sehr vorteilhaften Weise lassen sich mit der Erfindung auch Hautzellen bilden bzw. vermehren. In diesem Falle werden Bindegewebszellen der Haut z.B. mit Kollagen in die Zellkultur 11a - wie in Fig. 3 dargestellt - eingebracht. In einer zweiten Phase werden die Keratinozyten, d.h. verhornende Zellen, die die Epidermis ausmachen, aufgebracht, so daß das ganze mehrschichtig nach innen wächst. Nach Abschluß des Kulturverfahrens kann man die Folie aufschneiden oder abziehen. Damit eine Sterilität gegeben ist, kann man die Struktur auch in ein Gehäuse geben oder als kleine geschlossene Box ausbilden. Auf diese Weise läßt sich kontrolliert Haut züchten.

Im Unterschied zum Stand der Technik erfolgt dabei der Aufbau der Haut in einer Weise, wie sie später aufgebracht werden muß. Dies bedeutet, die Verhornung erfolgt außen und nicht innen, wobei man in diesem Falle nicht zuerst das Bindegewebe herstellt, sondern zuerst die Epidermis-Zellen direkt auf die Folie aufbringt und anschließend das Kollagen injiziert.

Bei der erfindungsgemäßen Vorrichtung können auch embryonale Zellen verwendet werden. Embryonale Zellen sind Zellen, die noch nicht ihre eigene Gewebsexpressionsmuster entwickelt haben. Durch die Mikroumgebung und die Matrix besteht dann die Möglichkeit, daß diese Zellen in eine Gewebsexpression übergeführt werden, die der terminalen Differenzierung, d.h. der endgültigen Differenzierung entspricht. Eine Beschichtung kann mit Peptiden erfolgen.

Als Folien können PTFE, Silikon, Polylaktid, Polyhydroxyalkanoat und Polyhydroxydbutyrate verwendet werden. Wesentlich ist, daß sie ganz allgemein zu der Gruppe der biodegradablen Polymere gehören, d.h. Strukturen, die entweder hydrolytisch oder enzymatisch abgebaut werden können, womit diese implantierbar sind.

Ein wesentliches Merkmal aller Trägerstrukturen bzw. der verschiedenen Ausgestaltungen der Träger 1 ist, daß sie formbar bzw. modellierbar sein können. Dadurch können neben Volumenveränderungen des Systems bzw. des Zellkulturraumes auch die Geometrie des Bioreaktors bzw. der Vorrichtung verändert werden. Das System kann auch im Betrieb durch Perfusionsdruckänderungen aufgrund der elastischen Eigenschaften der verwendeten Materialien in der Form reversibel verändert werden (Memory-Effekt).

In den Zellkulturraum 11a können Zellen 11 mit Matrix eingebracht werden, die durch den Zellkulturraum 11a modellierbar sind.

## Patentansprüche

1. Vorrichtung zum Züchten und/oder Behandeln von Zellen mit folgenden Merkmalen:
1.1 auf einem Träger (1) ist ein formbarer Zellkulturraum (11a) angeordnet, der durch den Träger (1) auf einer Seite oder eine auf den Träger (1) gelegte Trägerfolie (3) und eine darüber angeordnete Zellkulturfolie (2) auf der anderen Seite gebildet ist,
1.2 die Zellkulturfolie (2) ist derart elastisch, dass das Endvolumen des Zellkulturraumes (11a) ein Vielfaches seines Ausgangsvolumens einnehmen kann,
1.3 der Zellkulturraum (11a) ist mit jeweils einer Zu- und Ablaufleitung (4,10) versehen und
1.4 über den Träger (1) oder die Trägerfolie (3) und/oder die Zellkulturfolie (2) ist Sauerstoff oder Luft in den Zellkulturraum (11a) einbringbar,
**dadurch gekennzeichnet, dass**
der Träger (1) zur Anpassung des Zellkulturraums (11a) an die spätere Anwendungsform formbar ist.

2. Vorrichtung nach Anspruch 1, **dadurch gekennzeichnet, dass** der Zellkulturraum (11a) durch eine Struktur gebildet ist, die in ihrer Form wenigstens annähernd der späteren Anwendungsform entspricht.

3. Vorrichtung nach Anspruch 2,
**dadurch gekennzeichnet , dass**
die Struktur mikroporöse Zellen aufweist.

4. Vorrichtung nach Anspruch 2,
**dadurch gekennzeichnet, dass**
die Struktur biodegradabel ist.

5. Vorrichtung nach Anspruch 2,
**dadurch gekennzeichnet, dass**
die Struktur aus Kunststoff gebildet ist, der nach dem Züchten und/oder Behandeln der Zellen wieder entfernbar ist.

6. Vorrichtung nach Anspruch 1,
**dadurch gekennzeichnet, dass**
der Träger (1) mit der Zulauf- und der Ablaufleitung (4,10) versehen ist, die jeweils mit in der Oberseite des Trägers (1) angeordneten Rinnen (5,6,8) zur Verteilung einer Zellkultur (11) in dem Zellkulturraum (11a) in Verbindung stehen.

7. Vorrichtung nach Anspruch 6,
**dadurch gekennzeichnet, dass**
sich von einer Zulaufrinne (5) aus eine Querrinne (6) wenigstens annähernd über die Breite des Zellkulturraumes (11a) erstreckt, durch die eine wenigstens annähernd gleichmäßige Verteilung der Zellkultur (11) und ein wenigstens annähernd gleichmäßiger Fluß durch den Zellkulturraum (11a) erfolgt.

8. Vorrichtung nach Anspruch 7,
**dadurch gekennzeichnet, dass**
die Querrinne (6) mit zur Oberseite des Trägers (1) führenden Zungen (7) versehen ist, deren Längen mit zunehmendem Abstand von der Zulaufrinne (5) aus in ihrer Erstreckung und/oder ihrer Breite zunehmen.

9. Vorrichtung nach einem der Ansprüche 7 oder 8,
**dadurch gekennzeichnet, dass**
sich eine Ablaufrinne (8) in dem Träger (1) in Ablaufrichtung verbreitert.

10. Vorrichtung nach einem der Ansprüche 1 bis 9,
**dadurch gekennzeichnet, dass**
der Träger (1) und/oder die Trägerfolie (3) und/ oder die Zellkulturfolie (2) auf ihren zu dem Zellkulturraum (11a) gerichteten Seiten mit Strukturierungen versehen sind.

11. Vorrichtung nach Anspruch 10,
**dadurch gekennzeichnet, dass**
die Strukturierung durch Vertiefungen (12) gebildet sind.

12. Vorrichtung nach Anspruch 11,
**dadurch gekennzeichnet, dass**
zwischen den Vertiefungen (12) Wände (13) mit Durchbrechungen versehen sind.

13. Vorrichtung nach Anspruch 12,
**dadurch gekennzeichnet, dass**
die Vertiefungen (12) und die Wände (13) eine Art Labyrinth bilden.

14. Vorrichtung nach einem der Ansprüche 1 bis 13,
**dadurch gekennzeichnet, dass**
über der Zellkulturfolie (2) eine Abdeckplatte (17) angeordnet ist.

15. Vorrichtung nach Anspruch 14,
**dadurch gekennzeichnet, dass**
die Abdeckplatte (17) auf der zu der Zellkulturfolie (2) zugewandten Seite mit Kanälen und/oder Aussparungen (18) versehen ist.

16. Vorrichtung nach Anspruch 15,
**dadurch gekennzeichnet, dass**
die Abdeckplatte (17) an ihren Rändern luftdicht abgeschlossen ist und die Kanäle oder Bohrungen (18) mit einer Luft- oder Sauerstoffzuführung versehen sind.

17. Vorrichtung nach einem der Ansprüche 1 bis 16,
**dadurch gekennzeichnet, dass**
der Träger (1) über dem Zellkulturraum (11a) angeordnet ist, und dass die Zellkultur (11) im Anwachsstadium an der Zellkulturfolie (2) adhäriert ist.

18. Vorrichtung nach einem der Ansprüche 1 bis 17,
**dadurch gekennzeichnet, dass**
ein rahmenartiger Träger (1a) vorgesehen ist.

19. Vorrichtung nach einem der Ansprüche 1 bis 18,
**dadurch gekennzeichnet, dass**
der Träger (1,1a,1b) in Röhrenform oder Spiralform geformt ist.

20. Vorrichtung nach Anspruch 19,
**dadurch gekennzeichnet, dass**
der Träger (1) mit stirnseitigen Trägerringen (1b) versehen ist, zwischen denen der Zellkulturraum (11a) gebildet ist.

21. Vorrichtung nach Anspruch 19 oder 20,
**dadurch gekennzeichnet, dass**
der Zylindermantel des röhrenförmigen Zellkulturraumes (11a) durch die Trägerfolie (3) und die Zellkulturfolie (2) gebildet ist.

22. Vorrichtung nach einem der Ansprüche 1 bis 21,
**dadurch gekennzeichnet, dass**
in den Zellkulturraum (11a) Zellen mit Matrix eingebracht sind, die durch den Zellkulturraum (11a) modellierbar sind.

23. Vorrichtung nach Anspruch 1,
**dadurch gekennzeichnet, dass**
die Struktur wenigstens annähernd die Form einer Herzklappe, einer Blase oder einem Gefäß entspricht.

24. Vorrichtung nach Anspruch 23,
**dadurch gekennzeichnet, dass**
in den Zellkulturraum (11a) Bindegewebszellen eingebracht sind.

25. Vorrichtung nach Anspruch 19,
**dadurch gekennzeichnet, dass**
in die Röhrenform des Zellkulturraumes (11a) Einstülpungen (20) eingebracht sind.

26. Vorrichtung nach Anspruch 23,
**dadurch gekennzeichnet, dass**
die Einstülpungen (20) durch Klammern (22) gebildet sind.

27. Vorrichtung nach Anspruch 25 oder 26,
**dadurch gekennzeichnet, dass**
die Einstülpungen (20) mit Stabilisierungsringen (21) versehen sind.

28. Vorrichtung nach einem der Ansprüche 1 bis 27,
**dadurch gekennzeichnet, dass**
die Struktur durch eine Spritzgussform gebildet ist.

29. Vorrichtung nach einem der Ansprüche 1 bis 28,
**dadurch gekennzeichnet, dass**
die Struktur entfernbar oder auflösbar ausgebildet ist.

30. Vorrichtung nach einem der Ansprüche 1 bis 29,
**dadurch gekennzeichnet, dass**
der Träger (1) oder die Trägerfolie (3) und die Zellkulturfolie (2) mit der dazwischenliegenden Zellkulturkammer (11a) in einem Behälter (23) angeordnet ist, wobei sich in dem Behälter (23) Nährmedium befindet.

31. Vorrichtung nach Anspruch 30,
**dadurch gekennzeichnet, dass**
der Behälter (23) mit einem Deckel (24) versehen ist.

32. Vorrichtung nach Anspruch 30 und 31,
**dadurch gekennzeichnet, dass**
der Behälter mit einem Rührorgan (25) versehen ist.

33. Vorrichtung nach einem der Ansprüche 30 bis 32,
**dadurch gekennzeichnet, dass**
in dem Behälter (23) ein ringförmiger Zellkulturraum (11a) eingebracht ist, der auf einer Seite von der Trägerfolie (3), welche luftdurchlässig ist, und der auf der anderen Seite durch die Zellkulturfolie (2), die mikroporös ausgebildet ist, umgeben ist.

34. Vorrichtung nach einem der Ansprüche 1 bis 33,
**dadurch gekennzeichnet, dass**
mehrere Einheiten in einem Bioreaktor (14), der in Form eines Hochregales ausgebildet ist, angeordnet sind.

35. Vorrichtung nach Anspruch 34,
**dadurch gekennzeichnet, dass**
das Hochregal (14) mit einer Reihe von Einschubfächern (15) versehen ist, in die die Einheiten eingeschoben sind.

36. Vorrichtung nach Anspruch 34 oder 35,
**dadurch gekennzeichnet, dass**
der Bioreaktor (14) allseits geschlossen ist.

37. Vorrichtung nach Anspruch 34, 35 oder 36,
**dadurch gekennzeichnet, dass**
der Bioreaktor (14) mit Rädern (16) versehen ist.

38. Verfahren zum Züchten und/oder Behandeln von Zellen in einer Vorrichtung nach einem oder mehreren der Ansprüche 1 bis 37,
**dadurch gekennzeichnet, dass**
in einem ersten Schritt eine Matrix oder Kollagen in den Zellkulturraum (11a) injiziert wird, wobei bereits der Zellkulturraum (11a) mit Zellen (11) vorbeschichtet worden ist oder die Zellen (11) zusammen mit dem Kollagen oder der Matrix injiziert werden, in einem zweiten Schritt die Zellvermehrung stattfindet, wobei die Zellen (11) die Matrix oder das Kollagen umbauen und in einem dritten Schritt weitere Zelltypen zugegeben werden, wenn sich die Trägerfolie (3) und/ oder die Zellkulturfolie (2) aufgelöst hat/haben oder diese entfernt worden ist/sind.

39. Verfahren nach Anspruch 38,
**dadurch gekennzeichnet, dass**
vor oder nach Beendigung der Zellvermehrung die gesamte Struktur eingefroren wird.

## Claims

1. A device for culturing and/or treating cells, having the following features:
1.1 a shapable cell culture chamber (11a) is arranged on a support (1), which chamber (11a) is formed by the support (1) on the one hand or a support film (3) positioned on the support (1) and a cell culture film (2) arranged thereover on the other hand,
1.2 the cell culture film (2) is resilient in such a way that the final volume of the cell culture chamber (11a) may constitute a multiple of its starting volume,
1.3 the cell culture chamber (11a) is provided with in each case an inflow line and an outflow line (4, 10) and
1.4 oxygen or air may be introduced into the cell culture chamber (11a) via the support (1) or the support film (3) and/or the cell culture film (2),
**characterised in that**
the support (1) is shapable, for adapting the cell culture chamber (11a) to the subsequent application shape.

2. A device according to claim 1,
**characterised in that**
the cell culture chamber (11a) is formed by a structure which corresponds in shape at least approximately to the subsequent application shape.

3. A device according to claim 2,
**characterised in that**
the structure comprises microporous cells.

4. A device according to claim 2,
**characterised in that**
the structure is biodegradable.

5. A device according to claim 2,
**characterised in that**
the structure is formed of plastics, which may be removed again after culturing and/or treatment of the cells.

6. A device according to claim 1,
**characterised in that**
the support (1) is provided with the inflow and outflow lines (4, 10) which are in each case connected with conduits (5, 6, 8) arranged in the top of the support (1) for distributing a cell culture (11) in the cell culture chamber (11a).

7. A device according to claim 6,
**characterised in that**
there extends from an inflow conduit (5), at least approximately over the width of the cell culture chamber (11a), a transverse conduit (6) by means of which the cell culture (11) is at least approximately uniformly distributed and at least approximately uniform flow through the cell culture chamber (11a) is achieved.

8. A device according to claim 7,
**characterised in that**
the transverse conduit (6) is provided with tongues (7) leading to the top of the support (1), the lengths of which tongues increase in extent and/or width, the further they are from the inflow conduit (5).

9. A device according to one of claims 7 or 8,
**characterised in that**
an outflow conduit (8) in the support (1) widens in the outflow direction.

10. A device according to any one of claims 1 to 9,
**characterised in that**
the support (1) and/or the support film (3) and/or the cell culture film (2) are textured on their sides directed towards the cell culture chamber (11a).

11. A device according to claim 10,
**characterised in that**
the texturing takes the form of recesses (12).

12. A device according to claim 11,
**characterised in that**
walls (13) with openings are provided between the recesses (12).

13. A device according to claim 12,
**characterised in that**
the recesses (12) and the walls (13) form a type of labyrinth.

14. A device according to any one of claims 1 to 13,
**characterised in that**
a cover plate (17) is arranged over the cell culture film (2).

15. A device according to claim 14,
**characterised in that**
the cover plate (17) is provided with channels and/or cavities (18) on the side facing the cell culture film (2).

16. A device according to claim 15,
**characterised in that**
the cover plate (17) is sealed in airtight manner at its edges and the channels or bores (18) are provided with an air or oxygen feed.

17. A device according to any one of claims 1 to 16,
**characterised in that**
the support (1) is arranged above the cell culture chamber (11a) and **in that** the cell culture (11) is adhered to the cell culture film (2) in the growth stage.

18. A device according to any one of claims 1 to 17,
**characterised in that**
a frame-type support (1a) is provided.

19. A device according to any one of claims 1 to 18,
**characterised in that**
the support (1, 1a, 1b) is tubular or spiral in shape.

20. A device according to claim 19,
**characterised in that**
the support (1) is provided with support rings (1b) at the ends, between which the cell culture chamber (11a) is formed.

21. A device according to claim 19 or claim 20,
**characterised in that**
the cylindrical outer surface of the tubular cell culture chamber (11a) is formed by the support film (3) and the cell culture film (2).

22. A device according to any one of claims 1 to 21,
**characterised in that**
cells with matrix are introduced into the cell culture chamber (11a), these being mouldable by the cell culture space (11a).

23. A device according to claim 1,
**characterised in that**
the structure corresponds at least approximately to the shape of a heart valve, a bladder or a vessel.

24. A device according to claim 2,
**characterised in that**
connective tissue cells are introduced into the cell culture chamber (11a).

25. A device according to claim 19,
**characterised in that**
indentations (20) are introduced into the tubular shape of the cell culture chamber (11a).

26. A device according to claim 23,
**characterised in that**
the indentations (20) are formed by clips (22).

27. A device according to claim 25 or claim 26,
**characterised in that**
the indentations (20) are provided with stabilising rings (21).

28. A device according to any one of claims 1 to 27,
**characterised in that**
the structure is formed by an injection mould.

29. A device according to any one of claims 1 to 28,
**characterised in that**
the structure is removable or capable of disintegrating.

30. A device according to any one of claims 1 to 29,
**characterised in that**
the support (1) or the support film (3) and the cell culture film (2), with the cell culture chamber (11a) therebetween, is arranged in a container (23), nutrient medium being located in the container (23).

31. A device according to claim 30,
**characterised in that**
the container (23) is provided with a lid (24).

32. A device according to claim 30 and claim 31,
**characterised in that**
the container is provided with a stirring element (25).

33. A device according to any one of claims 30 to 32,
**characterised in that**
an annular cell culture chamber (11a) is introduced into the container (23), which chamber (11a) is surrounded on the one hand by the support film (3), which is air-permeable, and on the other hand by the cell culture film (2), which is of microporous construction.

34. A device according to any one of claims 1 to 33,
**characterised in that**
two or more units are arranged in a bioreactor (14), which takes the form of a rack unit.

35. A device according to claim 34,
**characterised in that**
the rack unit (14) is provided with a row of compartments (15), into which the units are inserted.

36. A device according to claim 34 or claim 35,
**characterised in that**
the bioreactor (14) is closed on all sides.

37. A device according to claim 34, 35, or 36,
**characterised in that**
the bioreactor (14) is provided with wheels (16).

38. A method of culturing and/or treating cells in a device according to one or more of claims 1 to 37,
**characterised in that**
in a first step a matrix or collagen is injected into the cell culture chamber (11a), wherein the cell culture chamber (11a) has already been precoated with cells or the cells (11) are injected together with the collagen or the matrix, in a second step cell proliferation takes place, wherein the cells (11) surround the matrix or the collagen, and in a third step further cell types are added, if the support film (3) and/or the cell culture film (2) has/have disintegrated or been removed.

39. A method according to claim 38,
**characterised in that**
the entire structure is frozen prior to or after completion of cell proliferation.

## Revendications

1. Dispositif pour la culture et/ou le traitement de cellules avec les caractéristiques suivantes:
1.1 un support (1) est constitué d'un espace de culture de cellules (11a) dont la forme est modifiable, qui est formé d'un côté du support (1) ou d'une feuille de support (3) qui est déposée sur ce support (1) et d'une feuille de culture de cellules (2) qui est déposée par-dessus, de l'autre côté du support,
1.2 la feuille de culture de cellules (2) est élastique de telle manière que le volume final de l'espace de culture de cellules (11a) peut être un multiple de son volume de sortie,
1.3 l'espace de culture de cellules (11a) est équipé d'un conduit d'amenée et d'un conduit d'écoulement (4,10), et
1.4 de l'oxygène ou de l'air peut être amené dans l'espace de culture des cellules (11a), au-dessus du support (1) ou de la feuille de support (3) et/ou de la feuille de culture de cellules (2),
**caractérisé en ce que,**
la forme du support (1) est modifiable en vue de l'adaptation de l'espace de culture de cellules (11a) à une forme d'utilisation ultérieure.

2. Dispositif selon la revendication 1,
**caractérisé en ce que,**
l'espace de culture de cellules (11a) est constitué d'une structure dont la forme correspond au moins approximativement à la forme d'utilisation ultérieure.

3. Dispositif selon la revendication 2,
**caractérisé en ce que**
la structure comprend des cellules microporeuses.

4. Dispositif selon la revendication 2,
**caractérisé en ce que**
la structure est biodégradable.

5. Dispositif selon la revendication 2,
**caractérisé en ce que**
la structure est réalisée en une matière synthétique qui peut être éloignée après la culture et/ou le traitement des cellules.

6. Dispositif selon la revendication 1,
**caractérisé en ce que**
le support (1) est équipé du conduit d'amenée et du conduit d'écoulement (4,10) qui sont respectivement en liaison avec des rigoles (5, 6, 8) ménagées sur le côté supérieur du support (1) pour la répartition d'une culture de cellules (11) dans l'espace de culture de cellules (11a).

7. Dispositif selon la revendication 6,
**caractérisé en ce que**
une rigole transversale (6) sortant d'une rigole d'amenée (5) s'étend au moins approximativement sur la largeur de la culture de cellules (11a), cette rigole permettant une répartition au moins approximativement uniforme de la culture des cellules (11) et un flux au moins approximativement uniforme à travers l'espace de culture des cellules (11a).

8. Dispositif selon la revendication 7,
**caractérisé en ce que**
la rigole transversale (6) est pourvue de languettes (7) orientées vers le haut du support (1), la longueur et/ou la largeur de ces languettes étant croissante en fonction de leur éloignement de la rigole d'amenée (5).

9. Dispositif selon l'une des revendications 7 ou 8,
**caractérisé en ce que**
une rigole d'écoulement (8) s'étend dans la direction de l'écoulement sur le support (1).

10. Dispositif selon l'une des revendications 1 à 9,
**caractérisé en ce que**
le support (1) et/ou la feuille de support (3) et/ou la feuille de culture des cellules (2) sont pourvues de structures sur leurs côtés orientés vers l'espace de culture des cellules (11a).

11. Dispositif selon la revendication 10,
**caractérisé en ce que**
la structuration est constituée de cavités (12).

12. Dispositif selon la revendication 11,
**caractérisé en ce que**
des parois (13) avec des passages sont ménagées entre les cavités (12).

13. Dispositif selon la revendication 12,
**caractérisé en ce que**
les cavités (12) et les parois (13) constituent une sorte de labyrinthe.

14. Dispositif selon l'une des revendications 1 à 13,
**caraactérisé en ce que**
une plaque de couverture (17) est disposée par-dessus la feuille de culture des cellules (2).

15. Dispositif selon la revendication 14,
**caractérisé en ce que**
la plaque de couverture (17) est pourvue, sur son côté orienté vers la feuille de cultures de cellules (2), de canaux et/ou d'évidements (18).

16. Dispositif selon la revendication 15,
**caractérisé en ce que**
la plaque de couverture (17) est fermée de façon étanche à ses bords et les canaux ou alésages (18) sont pourvus de conduits d'amenée d'air ou d'oxygène.

17. Dispositif selon l'une des revendications 1 à 16,
**caractérisé en ce que**
le support (1) est disposé au-dessus de l'espace de culture de cellules (11a), et **en ce que** la culture de cellules (11) adhère à la feuille de culture de cellules (2) dans sa phase de démarrage de la croissance.

18. Dispositif selon l'une des revendications 1 à 17,
**caractérisé en ce que**
le support se présente sous la forme d'un cadre (1a).

19. Dispositif selon l'une des revendications 1 à 18,
**caractérisé en ce que**
le support (1, 1a, 1b) a une forme tubulaire ou une forme spirale.

20. Dispositif selon la revendication 19,
**caractérisé en ce que**
le support (1) est pourvu d'anneaux de support frontaux (1b) entre lesquels est ménagé l'espace de culture de cellules (11a).

21. Dispositif selon la revendication 19 ou 20,
**caractérisé en ce que**
le manteau cylindrique de l'espace de culture de cellules (11a) de forme tubulaire est constitué de la feuille de support (3) et de la feuille de culture de cellules (2).

22. Dispositif selon l'une des revendications 1 à 21,
**caractérisé en ce que**
dans l'espace de culture de cellules (11a) sont introduites des cellules avec des matrices qui sont modelables par l'espace de culture de cellules (11a).

23. Dispositif selon la revendication 1,
**caractérisé en ce que**
la structure a au moins approximativement la forme d'une valve cardiaque, d'une vessie ou d'un réceptacle.

24. Dispositif selon la revendication 23,
**caractérisé en ce que**
des cellules de liaison tissées sont introduites dans l'espace de culture de cellules (11a).

25. Dispositif selon la revendication 19,
**caractérisé en ce que**
des plissages (20) sont ménagés dans l'espace de culture de cellules (11a).

26. Dispositif selon la revendication 23,
**caractérisé en ce que**
les plissages (20) sont réalisés au moyen de pinces (22).

27. Dispositif selon la revendication 25 ou 26,
**caractérisé en ce que**
les plissages (20) sont équipés de bagues de stabilisation (21).

28. Dispositif selon l'une des revendications 1 à 27,
**caractérisé en ce que**
la structure est réalisée par moulage par injection.

29. Dispositif selon l'une des revendications 1 à 28,
**caractérisé en ce que**
la structure est amovible ou soluble.

30. Dispositif selon l'une des revendications 1 à 29,
**caractérisé en ce que**
le support (1) ou la feuille de support (3) et la feuille de culture de cellules (2) avec la chambre de culture de cellules intercalaire (11a) sont disposés dans un récipient (23), ce récipient (23) contenant des substances nutritives.

31. Dispositif selon la revendication 30,
**caractérisé en ce que**
le récipient (23) est pourvu d'un couvercle (24).

32. Dispositif selon les revendications 30 et 31,
**caractérisé en ce que**
le récipient est pourvu d'un organe agitateur (25).

33. Dispositif selon l'une des revendications 30 à 32,
**caractérisé en ce que**
un espace de culture des cellules (11a) de forme annulaire est ménagé dans le récipient (23) et est entouré d'un côté par la feuille de support (3) qui est perméable à l'air et de l'autre côté par la feuille de culture des cellules (2) qui est microporeuse.

34. Dispositif selon l'une des revendications 1 à 33,
**caractérisé en ce que**
plusieurs unités sont disposées dans un bioréacteur (14), celui-ci ayant la forme d'une étagère placée en hauteur.

35. Dispositif selon la revendication 34,
**caractérisé en ce que**
l'étagère (14) est équipée d'une série de rayons coulissants (15) dans lesquels les unités sont engagées.

36. Dispositif selon la revendication 34 ou 35,
**caractérisé en ce que**
le bioréacteur (14) est fermé de tous les côtés.

37. Dispositif selon la revendication 34, 35 ou 36,
**caractérisé en ce que**
le bioréacteur (14) est pourvu de roues (16).

38. Procédé de culture et/ou de traitement de cellules dans un dispositif selon une ou plusieurs des revendications 1 à 37,
**caractérisé en ce que**,
au cours d'une première étape, une matrice de collagène est injectée dans l'espace de culture de cellules (11a), cet espace de culture de cellules (11a) ayant été préalablement garni de cellules (11) ou les cellules (11) étant injectées ensemble avec le collagène ou avec la matrice, au cours d'une deuxième étape s'effectue la multiplication cellulaire, les cellules (11) transformant la matrice ou le collagène et au cours d'une troisième étape, d'autres types de cellules sont ajoutés, lorsque la feuille de support (3) et/ou la feuille de culture des cellules (2) s'est/se sont dissoutes ou ont été éloignées.

39. Procédé selon la revendication 38,
**caractérisé en ce que**
l'ensemble de la structure est congelé avant ou après la fin de la multiplication cellulaire.
